# EUROPEAN PATENT APPLICATION

(11) **EP 2 003 447 A2**
(43) Date of publication of application: **17.12.2008**
(21) Application number: 07740305.3
(22) Date of filing: 29.03.2007
(51) Int. Cl.: G01N 27/416, G01N 27/30

(54) **METHOD FOR MEASURING PROTEIN**

(30) Priority: 29.03.2006 JP 2006092390
(71) Applicant: Horiba, Ltd., Kyoto-city, Kyoto 601-8510 (JP); Keio University, Tokyo 108-8345 (JP); Matsumoto, Koichi, Kyoto-city Kyoto 601-8510 (JP)
(72) Inventor: EINAGA, Yasuaki, Yokohama-shi, Kanagawa 223-8522 (JP); IVANDINI ANGGRANINGRUM, Tribidasari, Yokohama-shi, Kanagawa 223-8522 (JP); CHIKU, Masanobu, Yokohama-shi, Kanagawa 223-8522 (JP); YAMANUKI, Mikito, Kyoto-city Kyoto 601-8510 (JP)
(74) Representative: Hoffmann, Jörg Peter
(86) International application number: PCT/JP2007/056867
(87) International publication number: WO 2007/114252

(57) **Abstract**

The present claimed invention is to provide a method for measuring a protein that enables to detect the protein with high accuracy and high sensitivity through an electrochemical method at a high speed with a simple operation using a simple device. With the method, a working electrode 5 and a counter electrode 4 are made to contact with a sample solution 1 containing the protein as being an object to be measured, and a voltage is impressed between the working electrode 5 and the counter electrode 4 so that a current value at the voltage can be measured and the protein can be electrochemically analyzed.

## Description

### FIELD OF THE ART

This invention relates to a measuring method that can detect a protein with high accuracy and high sensitivity at a high speed by means of an electrochemical method with a simple operation using a simple device.

### BACKGROUND ART

Albumin is a protein that is contained the most in blood serum. Urine containing traces of albumin is called as micro albumin urine and is found in an early stage of diabetic nephropathy. Since it is important to take measures before the protein in urine shows a clear positive effect in order to prevent development of the diabetic nephropathy, it is extremely effective for early detection of the diabetic nephropathy to judge whether or not there is the micro albumin urine.

As a method for measuring an amount of albumin in urine, represented is a latex agglutination method, for example, as shown in patent document 1, with which albumin is measured by causing agglutination reaction with anti prealbumin and sensitized (bound) latex that is a bound antibody of anti prealbumin. More concretely, if a reagent used in the detection of albumin such as Albshua (a trade name manufactured by Eisai Co. Ltd.) as described in patent document 1 is used, an anti human albumin goat antibody is completely neutralized in case that albumin in urine exceeds 30 mg/L, albumin will not agglutinate even though albumin sensitized latex is added. In case that no albumin exists in urine or albumin in urine is less than 30 mg/L, albumin will agglutinate due to antigen antibody reaction with albumin sensitized latex because the anti human albumin goat antibody is left without being neutralized. A minute amount of albumin can be measured by making use of this property.
Patent document 1: Japan Patent Laid-open number H04-69572
Patent document 2: Japan Patent Laid-open number 2003-294679
Patent document 3: Japan Patent Laid-open number 2003-294680
Patent document 4: Japan Patent Laid-open number 2003-294681

### DISCLOSURE OF THE INVENTION

### PROBLEMS TO BE SOLVED BY THE INVENTION

However, with an immunity measuring method including the latex agglutination method, a reagent that contains an anti human albumin antibody is necessary. However, since the reagent that contains the anti human albumin antibody varies widely in quality, it is difficult to conduct a measurement stably with high accuracy and high sensitivity. In addition, a strict administration of the reagent is necessary in order to conduct a measurement stably with high accuracy and high sensitivity, and the strict administration of the reagent becomes a burden for medical institutions from both physical and human aspects.

Meanwhile, for example, as described in patent document 2 through patent document 4, there is a method for measuring an amount of albumin indirectly by the use of an enzyme-linked antibody wherein an electrochemically active enzyme is linked with an antibody of albumin as being an object to be measured by measuring an electrochemical reaction of the enzyme-linked antibody. However, since the electrochemical activity of albumin is not high, it has been considered that it is difficult to directly measure the albumin by the use of the electrochemical method. Furthermore, with the electrochemical method, there is also a concern about an adverse influence on the measurement because a protein is absorbed into a surface of the electrode.

In addition, since an immune suppression acid protein (hereinafter also called as IAP) as being an immunosuppressive agent found in blood of various kinds of tumor bearing patients shows an increase of a concentration at a high percentage for one kind of tumor bearing patients and a high value is expressed in compliance with an advanced clinical stage, the immune suppression acid protein can be a tumor marker that is nonspecific to an organ. Furthermore, it is also reported that IAP is used as an index for effectiveness prediction of an aid immune chemical therapy for malignant diseases from a view point of its immune suppression activity.

Meanwhile, C-reactive protein (hereinafter also called as a CRP) is a protein whose number increases at a time when inflammation or destruction of a living body is generated in a human body, and linked with C-polysaccharide of pneumococcal organism. Since a number of the CRP increases due to inflammation, the CRP can be used as a marker protein for various diseases. In addition, the CRP reacts perceptively to inflammation of blood vessels, it draws attention as a marker to vascular diseases such as myocardial infarction. Since myocardial infarction is difficult to predict, it is expected to establish a diagnostic method by the use of the CRP.

Contrary, the present claimed invention is to provide a measuring method that enables detection of various kinds of proteins at a high speed with high accuracy and high sensitivity by the use of the electrochemical method with a simple operation using a simple device.

### MEANS TO SOLVE THE PROBLEMS

More specifically, the method for measuring a protein in accordance with this invention is characterized by making a working electrode and a counter electrode contact with a sample solution containing a protein as being an object to be measured, impressing a voltage between the working electrode and the counter electrode, measuring an electric current value at this voltage and analyzing the protein electrochemically.

The method for measuring the protein in accordance with this invention was invented because the inventor had found that a high polymer substance such as a protein could be measured electrochemically if a conductive diamond electrode was used. Since the method for measuring the protein in accordance with this invention is an electrochemical analysis method, this method does not require a reagent that contains an antibody unlike an immunity measuring method that is based on an antigen-antibody reaction such as a conventional broadly used latex agglutination method. As a result, it is possible to analyze the protein stably with high accuracy and high sensitivity without a concern about falling a measurement accuracy and a measurement sensitivity due to fluctuation of the quality of the reagent that might occur in case of the immunity measuring method. In addition, since the reagent that contains an antibody is not necessarily required, an operation of analysis is extremely easy and a result of the analysis can be obtained at once. Furthermore, the analysis can be conducted also on a small amount of a sample and the measuring device can be downsized. With the method for measuring the protein in accordance with this invention, it is possible to detect the protein of low concentration with high sensitivity if a boron dope diamond electrode is used as a working electrode.

### EFFECT OF THE INVENTION

In accordance with this invention, it is possible to detect a protein at a high speed with high accuracy and high sensitivity with a simple operation using a simple device.

### BRIEF DESCRIPTION OF THE DRAWINGS

Fig. 1 is a voltamogram showing a result of a cyclic voltammetry measurement of bovine serum albumin as being an object to be measured.
Fig. 2 is a voltamogram showing a result wherein cathodic reduction is provided to a diamond electrode after the bovine serum albumin is measured.
Fig. 3 is a voltamogram showing a result of a cyclic voltammetry measurement of an immunosuppressive acid protein as being the object to be measured.
Fig. 4 is a voltamogram showing a result of a cyclic voltammetry measurement of hemoglobin as being the object to be measured.
Fig. 5 is a voltamogram showing a result of a cyclic voltammetry measurement of pepsin as being the object to be measured.
Fig. 6 is a voltamogram showing a result of a cyclic voltammetry measurement of C-reactive protein as being the object to be measured.
Fig. 7 is a general view of an electrochemical measuring device showing one embodiment of this invention.
Fig. 8 is a general view showing a structure of a flow cell of the electrochemical measuring device shown in Fig. 7.
Fig. 9 is a calibration curve showing a relationship between an albumin concentration (horizontal axis) and a current value (vertical axis) obtained by a flow injection measurement.
Fig. 10 is a voltamogram obtained by a cyclic voltammetry measurement obtained by linear sweeping (20mV/s) with changing a concentration of tetrodotoxin.
Fig. 11 is a calibration curve showing a relationship between a tetrodotoxin concentration (vertical axis) and a current value (horizontal axis) obtained by impressing a voltage that gives the maximum current.

### EXPLANATION OF THE CODE

- 1: sample solution
- 2: pump
- 3: flow cell
- 4: counter electrode
- 5: working electrode
- 6: reference electrode
- 7: potentiogalvanostat
- 8: information processing unit

### BEST MODES OF EMBODYING THE INVENTION

A protein as being an object to be measured in this invention is not limited. However, this invention is preferably applied to a high polymer protein whose molecular weight is more than or equal to 10,000, and, can measure, for example, albumin, IAP, pepsin, hemoglobin and CRP with high accuracy and high sensitivity. In addition, with this invention, it is possible to measure a natural poison such as tetrodotoxin.

The method for measuring protein in accordance with this invention uses a conductive diamond electrode or a carbon electrode as a working electrode.

An electrode having a diamond thin film that is made to be conductive by doping an element in group 13 or group 15 is represented as the conductive diamond electrode. Among them, it is preferable that at least one element selected from a group comprising boron, nitrogen and phosphorus is doped, and more preferably the conductive diamond electrode is a boron dope diamond electrode into which boron is doped.

In addition, an electrode having a diamond thin film that is made to be conductive by doping an element that belongs to either one of group 8, group 9 and group 10 can be used as the conductive diamond electrode. An electrode into which at least one kind of the element selected from the group comprising platinum, palladium, nickel, ruthenium and iridium is doped is represented as the conductive diamond electrode.

With the method for measuring a protein in accordance with this invention, in order to calculate a concentration of the protein as being the object to be measured in the sample solution from an obtained electric current value, a calibration curve prepared in advance showing a relationship between a concentration of the protein as being the object to be measured and the electric current value is compared with the obtained electric current value.

It is preferable that the voltage impressed between the working electrode and the counter electrode is a voltage that gives the maximum electric current value.

With the method for measuring the protein in accordance with this invention, in case that the working electrode is the conductive diamond electrode, a process to regenerate the conductive diamond electrode by the cathodic reduction may be further comprised.

Since diamond is resistant to every acid and base, it is possible to conduct a powerful clean such as a piranha clean on the conductive diamond electrode. However, it is not appropriate to use acid or base for cleaning from a viewpoint of risk or environmental burden, and it is preferable to use the cathodic reduction that has a less burden on environment as a method for regenerating the conductive diamond electrode whose surface is poisoned by oxidization of the protein.

In order to regenerate the conductive diamond electrode by means of the cathodic reduction, a high negative voltage is impressed to the conductive diamond electrode so as to generate hydrogen from a surface of the electrode by means of electrolysis of water by the use of aqueous solution into which, for example, an electrolyte is dissolved.

In case of a metal electrode, when hydrogen is generated, the electrode is dissolved or inactivated depending on a condition such as pH. Meanwhile, in case of a glassycarbon electrode (hereinafter also called as a GC electrode), the electrode is physically destructed due to generation of hydrogen. However, in case of the conductive diamond electrode, neither physical destruction nor chemical destruction occurs due to generation of hydrogen.

A mechanism to remove the attached protein from the conductive diamond electrode by means of generation of hydrogen is as follows.
(1) A surface of an electrode can be made acid environment irrespective of a pH of a solution due to generation of hydrogen, and the protein is decomposed under the acid environment.
(2) A chemically active hydrogen radical is generated at a time of generation of hydrogen, and the protein is chemically decomposed by the hydrogen radical.
(3) Bubbles are generated at a time when gaseous hydrogen is generated at normal temperature and normal pressure, and the protein is physically peeled off.

With the above three mechanisms, it becomes possible to regenerate the conductive diamond electrode by means of the cathodic reduction.

In case that the object to be measured is a low-molecular weight protein whose molecular weight is about 6000 and below such as insulin, a sensitivity of the conductive diamond electrode is deteriorated due to absorption of the protein after a measurement is conducted three or four times. However, in case that the object to be measured is a high molecular protein whose molecular weight is over 10000 such as CRP, a sensitivity of the conductive diamond electrode is deteriorated after one measurement. This is conceivably resulted from that the bigger the molecular weight is, the more significantly the protein is absorbed. In addition, in case that the object to be measured is the low-molecular protein such as insulin, it is possible for the cathodic reduction to regenerate the conductive diamond electrode to regenerate the conductive diamond electrode with a relatively low voltage of about 1V. In case that the object to be measured is the high molecular protein such as CRP, a high voltage of about 3V is necessary for the cathodic reduction to regenerate the conductive diamond electrode.

The method for measure the protein in accordance with this invention may be a double electrode method using a working electrode and a counter electrode. In case of conducting a measurement with high sensitivity and high accuracy, a triple electrode method further using a reference electrode is preferable, and an absolute value of the voltage impressed between the working electrode and the counter electrode may be controlled by making the reference electrode contact with a sample solution that contains a protein as being the object to be measured.

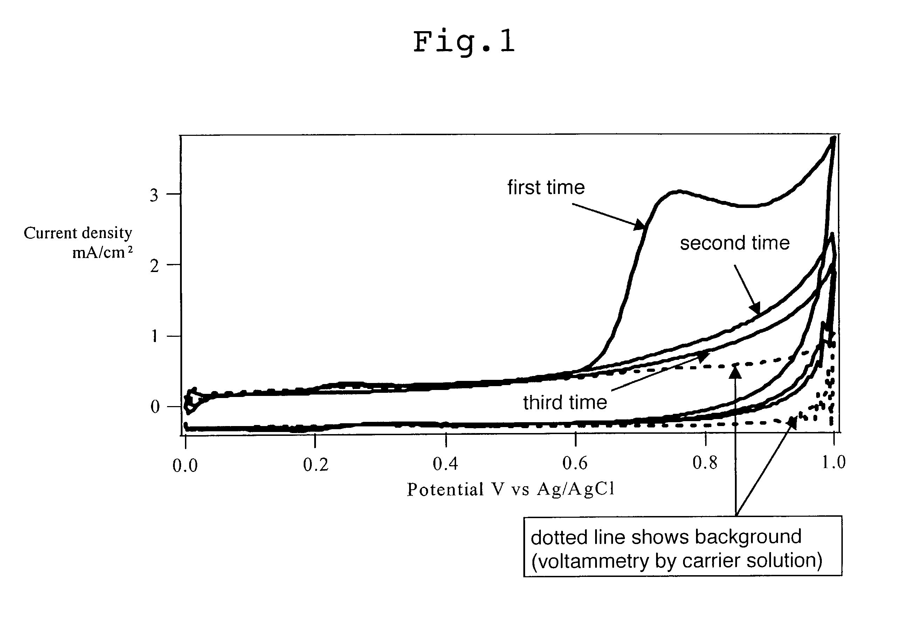

Fig. 1 is a cyclic voltamogram showing a result at pH7 among the results of the cyclic voltammetry measurement under the following condition with the bovine serum albumin (hereinafter also called as BSA) used as the object to be measured, and with an boron dope diamond electrode used as the working electrode.
Sample solution; 300 mg/dl BSA (0.1 M phosphate buffer solution (PBS) pH2, 7, 10)
Reference pole; Ag/AgCl
Antipole; platinum
Potentiostat; HSV-100 (manufactured by Hokuto Denko Corporation)

A dotted line in Fig. 1 shows a background in PBS as being a carrier solution.

As shown in Fig. 1, although a clear oxidization peak was confirmed at around 0.75V, an oxidization current became smaller each cycle. This was conceivably resulted from that the BSA was absorbed into a surface of the boron dope diamond electrode.

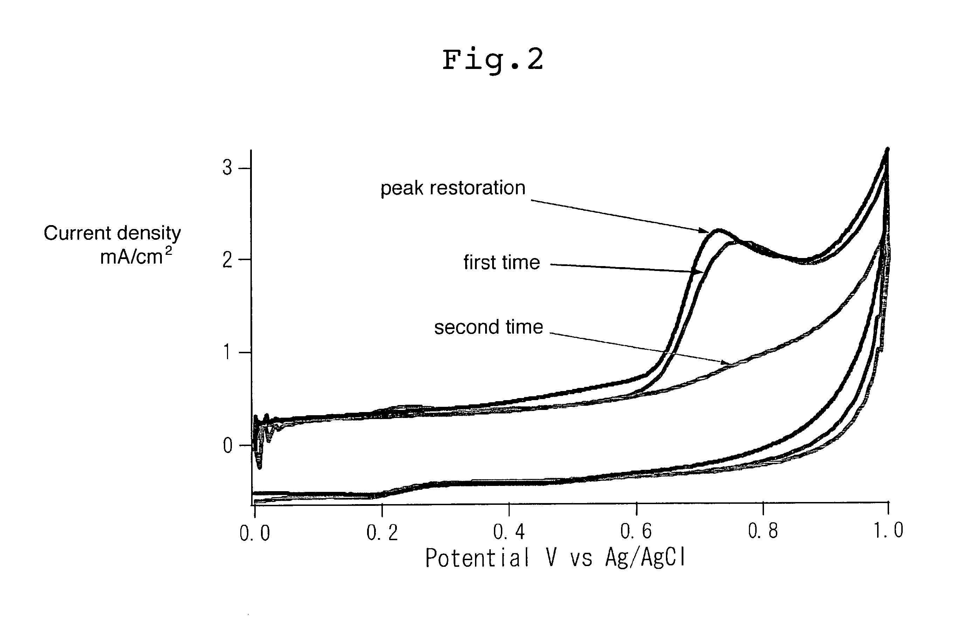

Then we tried to regenerate the electrode by providing the diamond electrode after the BSA was measured with the cathodic reduction. When the cathodic reduction was conducted under the condition of -3V, 10 min with the solution of 1M PBS, it was revealed that the activity of the electrode was restored as shown in Fig. 2.

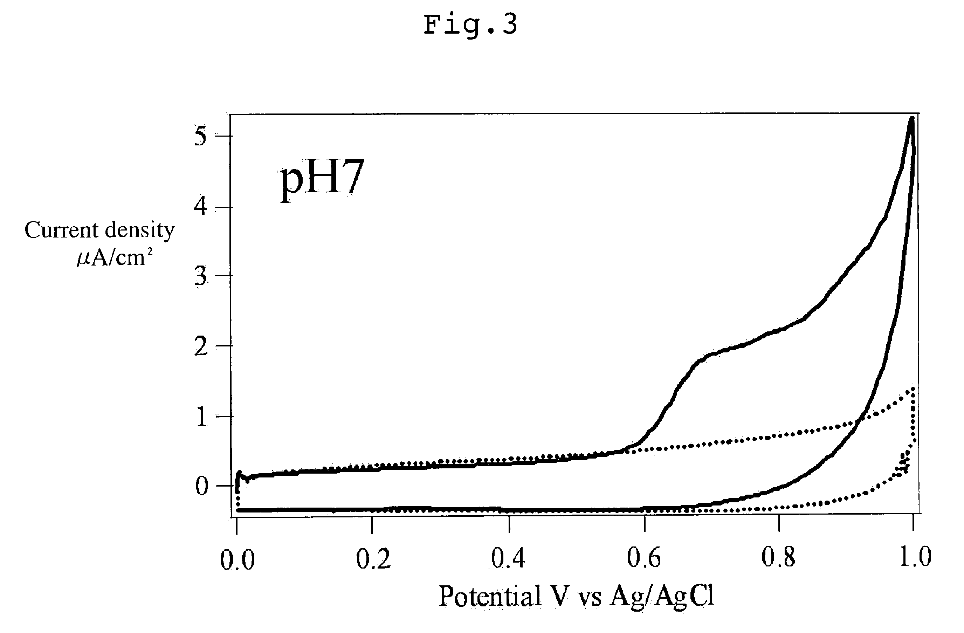

Furthermore, when an electrochemical measurement was conducted with IAP being as the object to be measured, the oxidization peak like the BSA was obtained at pH7 as shown in Fig. 3.

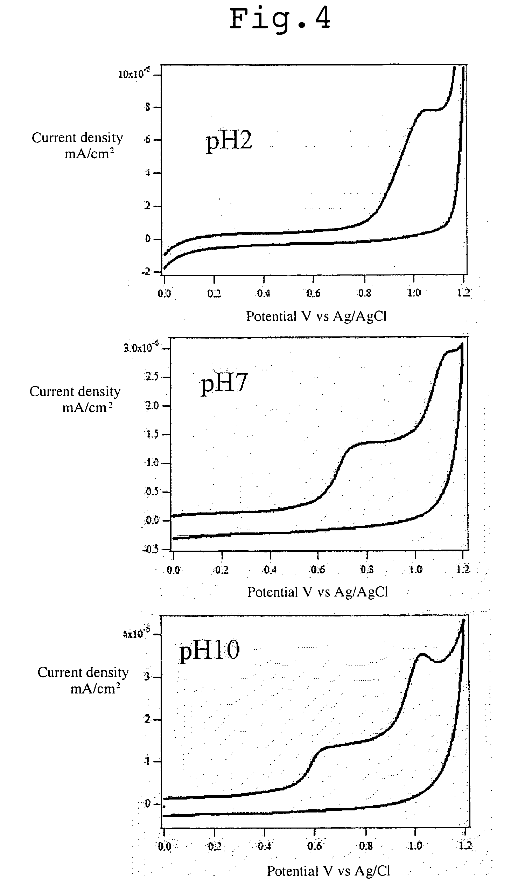

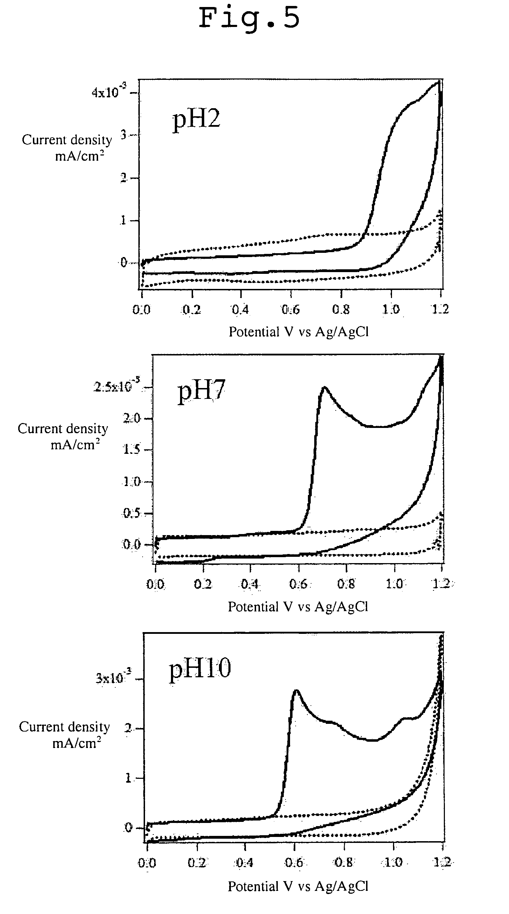

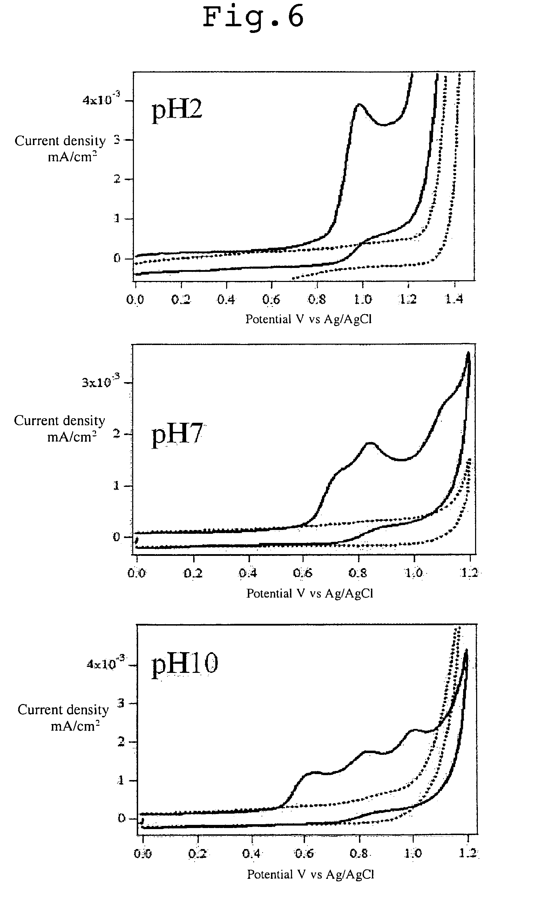

Additionally, when the cyclic voltammetry measurement was conducted on three kinds of the proteins; hemoglobin, pepsin and CRP under the condition of pH2, 7 and 10 similar to the BSA, clear oxidization peaks were confirmed as shown in Figs. 4, 5 and 6.

A method for measuring a protein in accordance with this invention can be embodied by, for example, a measuring device having the following arrangement. More specifically, the device is a measuring device for detecting a protein as being an object to be measured in a sample solution, and comprises a cell that incorporates a working electrode and a counter electrode, a device that impresses a voltage to the working electrode and the counter electrode, a device that measures an electric current value at the impressed voltage, and an information processing unit that detects the protein based on the obtained electric current value.

For the measuring device, it is preferable that the working electrode is a conductive diamond electrode, and more preferably a boron dope diamond electrode.

In addition, the measuring device may have an arrangement wherein the cell further incorporates a reference electrode, and a device that controls an absolute value of the voltage impressed between the working electrode and the counter electrode may be further comprised.

When a measurement is conducted, the protein is absorbed into the diamond electrode and the diamond electrode loses the ability as an electrode. However, in this case, it is possible to regenerate the electrode by removing the absorbed protein with conducting the cathode reduction. The measuring device in accordance with this invention may be so arranged that the conductive diamond electrode is regenerated by means of the cathodic reduction.

In addition, a protein may be analyzed electrochemically with processes of making a carrier solution at a constant flow rate contact with a working electrode, injecting the protein as being an object to be measured into the carrier solution, making the carrier solution contact with the working electrode and a counter electrode, impressing a voltage between the working electrode and the counter electrode, and measuring an electric current value at this voltage. With this arrangement, the protein is difficult to be absorbed into a surface of the electrode, which makes it possible to conduct a measurement continuously. Furthermore, since a zone of a concentration on which the measurement can be conducted is close to a human blood concentration, this method becomes preferable.

One embodiment of the electrochemical measuring method used for this invention will be explained with reference to drawings.

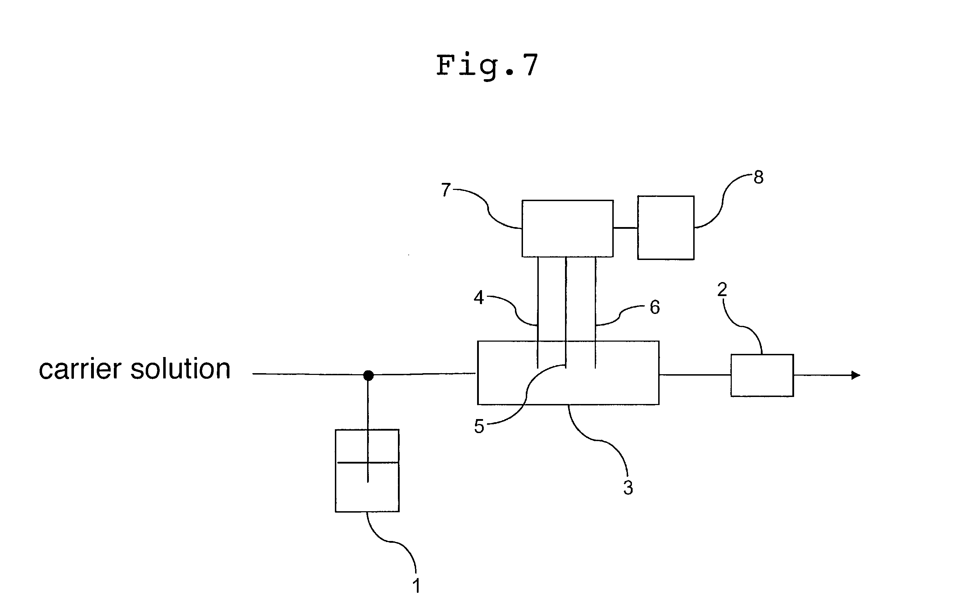

Fig. 7 shows one embodiment of the device to embody the method for measuring the protein in accordance with this invention, and shows a general view of an electrochemical measuring device (a flow injection analytical device) of a liquid injection type using a flow cell for electrochemical measurement.

With a flow injection analysis, a continuous flow controlled by a constant rate pump is created, and various reaction, separation or injection of the sample is conducted in this flow so as to analyze a component in the sample solution by the use of a detector comprising a flow cell arranged at a distal end.

In the electrochemical measuring device shown in Fig. 7, a flow cell 3 into which a counter electrode 4, a working electrode 5 and a reference electrode 6 are incorporated, and a pump 2 are arranged from upstream of a flow channel, and the counter electrode 4, the working electrode 5 and the reference electrode 6 are connected to a potentiogalvanostat 7 and an information processing unit 8 is connected to the potentiogalvanostat 7.

In case of measuring a concentration of the protein as being the object to be measured by the use of the electrochemical measuring device shown in Fig. 7, first a sample solution 1 containing the protein as being the object to be measured is injected into a carrier solution such as PBS that is flowing in a flow path so as to be sent to the flow cell 3 by the pump 2. In the flow cell 3, an electrochemical reaction occurs by impressing a voltage between the working electrode 5 and the counter electrode 4 in a state the incorporated counter electrode 4, working electrode 5 and reference electrode 6 contact with the sample solution 1. An electric current value (electric signal) generated by the electrochemical reaction is transmitted to the potentiogalvanostat 7 and then the signal at each electrode is controlled and detected. The signal detected by the potentiogalvanostat 7 is analyzed by the information processing unit 8 so as to detect each protein and to measure its concentration. The sample solution 1 after completion of the measurement is discharged outside of the electrochemical measuring device.

A solution containing a protein such as albumin, IAP, pepsin, hemoglobin and CRP can be represented as the sample solution 1. Among them, for example, whole blood, blood serum, blood plasma and urine can be represented as a solution containing albumin as being a marker for diabetic nephropathy. With the method for measuring albumin by the use of the electrochemical measuring device, it is possible to conduct a measurement without necessarily using a measuring reagent containing an anti-albumin antibody unlike a conventional immune measuring method utilizing an antigen-antibody reaction. As a result, it is possible to conduct the measurement stably with a high accuracy and a high sensitivity without a concern about degrading a measurement accuracy or a measurement sensitivity due to fluctuation of a quality of a measuring reagent. In addition, since it is also possible to conduct the measurement without using a measuring reagent containing the anti-albumin antibody, a structure of the measuring device and its operation can be simplified.

Furthermore, the electrochemical measuring device can also measure a sample solution 1 containing a natural poison such as tetrodotoxin as being a fugu poison. Tetrodotoxin induces paralysis of a nerve or a muscle by preventing transmission of nerve information because tetrodotoxin is combined with a sodium channel as being a sodium ion channel. With the measuring method in accordance with this invention, since whether there is tetrodotoxin or not can be measured with ease, it is possible to improve safety in case of eating puffer fish. For example, a solution wherein tissues of puffer fish in a paste form is suspended into a buffer solution can be represented as the sample solution 1 containing tetrodotoxin.

The pump 2 is not especially limited as long as it can send the sample solution 1 to the flow cell 3 at a constant rate, and may use a pump for, for example, liquid chromatography.

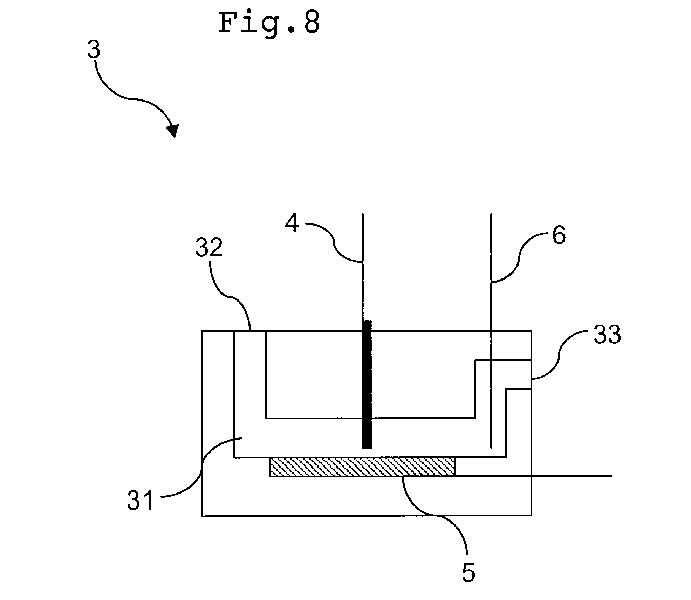

The flow cell 3 has an arrangement, as shown in Fig. 8, that the working electrode 5, the counter electrode 4 and the reference electrode 6 are exposed in a flow channel 31 where the sample solution 1 flows so as to contact the sample solution 1. In case that the working electrode 5 is the conductive diamond electrode, a diamond thin film of the conductive diamond electrode is exposed in the flow channel 31 and contacts the sample solution 1. The sample solution 1 enters from an inlet 32 of the flow channel 31 and flows in a direction shown by an arrow in Fig. 8 and then reaches an outlet 33. Then the electrochemical reaction is caused in the sample solution 1 by impressing a voltage between the working electrode 5 and the counter electrode 4. The working electrode 5 may be a micro electrode or a rotational electrode.

As the working electrode 5, a conductive diamond electrode or a carbon electrode may be used.

Diamond is originally a superior insulating body. However, the diamond shows electrical conductivity similar to semiconductor - metal by adding traces of an element in group 13 or group 15. In this invention, the diamond that shows electrical conductivity similar to the semiconductor - metal is used as the electrode.

As a substance to be doped in order to give the electrical conductivity to the diamond, an element in group 13 or group 15 is represented, and more preferably boron, nitrogen and phosphorus are represented, and further more preferably boron is represented. The boron dope diamond electrode doped with boron in a high concentration has a broad potential window (oxidation potential and reduction potential are broad) and has an advantageous characteristic that a background electric current is low compared with other substance for electrodes. In addition, the boron dope diamond electrode is superior in chemical resistance, durability, electrical conductivity and resistance to corrosion.

An additive amount of a substance doped into the diamond in order to give the conductivity to the diamond can be determined arbitrarily within a range wherein the conductivity can be given to the diamond, and is preferably an amount that can give, for example, about 1×10⁻² - 10⁻⁶ Ωcm of the conductivity, and the additive amount is conventionally controlled during a process of manufacturing.

The conductive diamond itself can be made to be an electrode without being supported by a substrate, however, it is preferable to form a thin film of the conductive diamond on a substrate and to connect electric leads to the thin film so as to be an electrode. Si (for example, single crystal silicon), Mo, W, Nb, Ti, Fe, Au, Ni, Co, Al₂O₃, SiC, Si₃N₄, ZrO₂, MgO, graphite, single crystal diamond, cBN and silica glass can be represented as the substrate, and especially single crystal silicon, Mo, W, Nb, Ti, SiC and single crystal diamond are preferably used.

A thickness of the conductive diamond thin film is not especially limited, and is preferably about 1 ∼ 100 µm, and more preferably about 5 ∼ 50 µm.

In this invention, the conductive diamond electrode may be in a form of a micro electrode. The conductive diamond electrode in the form of the micro electrode is made by cutting a distal end of a thin line made of Pt in a keen state, and by electrolytically polishing the distal end to be further keener, and then by forming a thin film of the conductive diamond on a surface of the distal end.

The conductive diamond thin film can be manufactured by the use of a chemical vapor deposition method. The chemical vapor deposition method is a method to synthesize a substance by chemically reacting the gaseous substance in the gas phase, and is generally called as CVD (Chemical Vapor Deposition) method. This method is widely used for a process of manufacturing semiconductors, and can be utilized for manufacturing the conductive diamond film in this invention if altered appropriately.

Chemical vapor synthesis of diamond uses a gas that is made by doping hydrogen and a gas containing carbon such as methane as a raw gas and the gas is excited by an excitation source and the excited gas is deposited on the substrate.

A thermo filament, a micro wave, a high frequency wave, a direct-current glow discharge, a direct-current arc discharge and a combustion flame can be represented as the excitation source. In addition, some of the excitation sources may be combined to adjust a nucleation density so as to enable a large area or homogenization.

Various compounds that contain carbon and that produce active carbon such as C and C₂, and a hydrocarbon radical such as CH, CH₂, CH₃, and C₂H₂, when it is decomposed and excited by an excitation source can be used as a raw material, and CH₄, C₂H₂, C₂H₄, CO, and CF₄ as gas, CH₃OH, C₂H₅OH, and (CH₃)₂CO as liquid, and graphite and fullerene as solid can be represented as a preferable example.

In the chemical vapor deposition method, a substance that gives the conductivity to the diamond may be added with a method wherein a disk of an additive substance is placed in a system and excited like the carbon source substance so that the additive substance is introduced into a carbon gas phase, or with a method wherein an additive substance is previously added to the carbon source and the carbon source added with the additive substance is introduced into a system and excited by the excitation source so that the additive substance is introduced into a carbon gas phase. The later method is preferable. Especially, in case of using a liquid such as acetone and methanol as the carbon source, a method wherein boron oxide (B₂O₃) is dissolved into the liquid so as to be a boron source is preferable because a concentration of boron can be easily and simply controlled with this method. In the chemical vapor deposition method, in case of adding boron to the carbon source, a concentration of boron is generally about 10 - 12,000 ppm, however, about 1,000 - 10, 000 ppm is preferable.

As the chemical vapor deposition method, a plasma chemical vapor deposition method is preferably used. With the plasma chemical vapor deposition method, an activation energy that causes a chemical reaction is high and the chemical reaction is quick. Furthermore, in accordance with this method, the chemical reaction can be caused at a low temperature by producing a chemical species that does not exist thermodynamically unless at a high temperature. The conductive diamond thin film by the plasma chemical vapor deposition method can be manufactured in accordance with a method described in, for example, Yano et al., J. Electrochem. Soc., 145 (1998) 1870.

The boron dope diamond electrode used in this invention can be manufactured by a manufacturing method using, for example, a micro wave plasma assist CVD method that will be explained next. A concrete manufacturing method is as follows. First, hydrogen plasma is produced in a chamber where hydrogen gas is filled, acetone and methanol mixed gas into which a boron species is dissolved is introduced into the chamber, and then a carbon source is further introduced into the chamber so that a gas phase is grown on a conductive (or semi-conductive) substrate such as, for example, a silicon substrate. A silicon substrate {Si(100)} is used as the substrate. After a surface of the substrate is texture-processed (for example, polished with 0.5 µm of diamond powders), the substrate is set on a holder of a film forming device. The acetone and methanol mixed substance (in a form of a liquid and its mixing ratio is 9:1 in volume) is used as a source for film forming, and boron oxide (B₂O₃) is dissolved into the mixed substance so that a ratio of boron/carbon (B/C) becomes 10⁴ ppm. After pure hydrogen (H₂) gas as a carrier gas is passed through the source for film forming, the gas is introduced into the chamber and then the pressure in the chamber is adjusted to be a predetermined pressure (for example, 115 Torr = 115 × 133.322 Pa) by passing hydrogen (for example, 532 ml/min) from another line. Then after a micro wave electric power of 2.45 GHz is injected and discharged, the electric power is adjusted to be 5kW. After stabilized, film forming is conducted by passing the pure hydrogen gas (for example, 15 ml/min) as the carrier gas through the source for film forming.

With the manufacturing method using the above-mentioned micro wave plasma assist CVD method, when film forming of the diamond thin film was conducted until a film thickness became about 30 µm at a film forming rate of 1 - 4 µm/hour, it was observed that a temperature of the substrate was about 850 - 950 °C in a steady state although no heating was applied to the substrate. In addition, only a single peak was observed at 1333 cm⁻¹ of a raman spectrum of the obtained diamond thin film. Furthermore, electric conductivity was about 10⁻³ Qcm, and -1.25 - +2.3 V (to SHE (standard hydrogen electrode)) and having a broad potential window were confirmed as a result of measuring cyclic voltamogram in 0.5 M sulfuric acid.

In case of using the conductive diamond electrode as the working electrode 5, an electrode having a diamond thin film that is made to be conductive by doping an element belonging to either one of group 8, group 9 and group 10 may be used. As the above-mentioned element, platinum, palladium, nickel, ruthenium and iridium are preferably used.

In order to introduce the element into diamond, an ion implantation method can be used. A metal - diamond composite electrode manufactured by the ion implantation method is high in stability so that the metal is difficult to be detached even though ultrasonic cleaning is conducted, and is superior in metal dispersibility.

In case of using a carbon electrode as the working electrode 5, for example, a glassy carbon electrode is preferably used. The glassy carbon electrode has the characteristics of being high in electric conductivity, stable against chemical agency, having the same properties of being incapable of passing a gas as that of thermal decomposition graphite, inexpensive and relatively large in overvoltage against generation of hydrogen or oxygen.

For example, platinum, carbon, stainless, gold, diamond and SnO₂ can be used as the counter electrode 4.

The voltage impressed between the working electrode 5 and the counter electrode 4 is preferably a voltage that gives the maximum current value from a view point of a measurement efficiency and a measurement accuracy. The voltage that gives the maximum current value is a voltage that gives the maximum current value by, for example, the cyclic voltammetry. In addition, the voltage that gives the maximum current value can be obtained by the rotational electrode method or the micro electrode method. The rotational electrode method or the micro electrode method is advantageous in a point that a possibility of a measurement error depending on a measurement condition can be further eliminated.

A well-known electrode may be utilized as the reference electrode 6, and a standard hydrogen electrode, a silver/silver chloride electrode, a mercury/mercurous chloride electrode, a hydrogen palladium electrode or the like may be used as the reference electrode 6.

The electrochemical measuring device conducts a measurement by the use of a triple electrode method comprising the counter electrode 4, the working electrode 5 and the reference electrode 6. The electrochemical measuring device to conduct the measuring method in accordance with this invention may use a double electrode method comprising the working electrode 5 and the counter electrode 4 alone. Since the triple electrode method can control an absolute value of the voltage impressed between the working electrode 5 and the counter electrode 4, it is possible to conduct a measurement with high accuracy and high sensitivity. Meanwhile, with the double electrode method, since two kinds of the electrode, only the working electrode 5 and the counter electrode 4 will do, a structure of the flow cell 3 can be simplified and downsized and the measurement cell can be formed in chip so as to be disposable, thereby enabling further more simple measurement.

In measuring the protein, first, only a carrier solution that contains no protein as being an object to be measured is flown into the flow cell 3 so as to lessen and stabilize, so called, a background electric current as much as possible. The diamond electrode is characterized by that its background electric current is small. Next, the sample solution 1 is mixed with the carrier solution and the mixed solution is flown into the flow cell 3. A flow pass through which the carrier solution flows into the flow cell 3 is not drawn in the drawings.

Next, the electric voltage is impressed between the working electrode 5 and the counter electrode 4 so that the protein as being the object to be measured is oxidized or reduced and the electric current accompanied by the oxidation-reduction reaction is generated. The electric current (electric signal) is transmitted to the potentiogalvanostat 7 and the signal detected by the potentiogalvanostat 7 is analyzed by the information processing unit 8.

The information processing unit 8 has a CPU, an internal memory, an external memory device such as an HDD, a communication interface such as a modem, a display and an input device such as a mouse or a keyboard. And the information processing unit 8 analyzes the electric signal based on a program stored in a predetermined area of the internal memory or the external memory device and detects the protein or calculates the concentration of the protein. The information processing unit 8 may be a general-purpose computer or a dedicated computer.

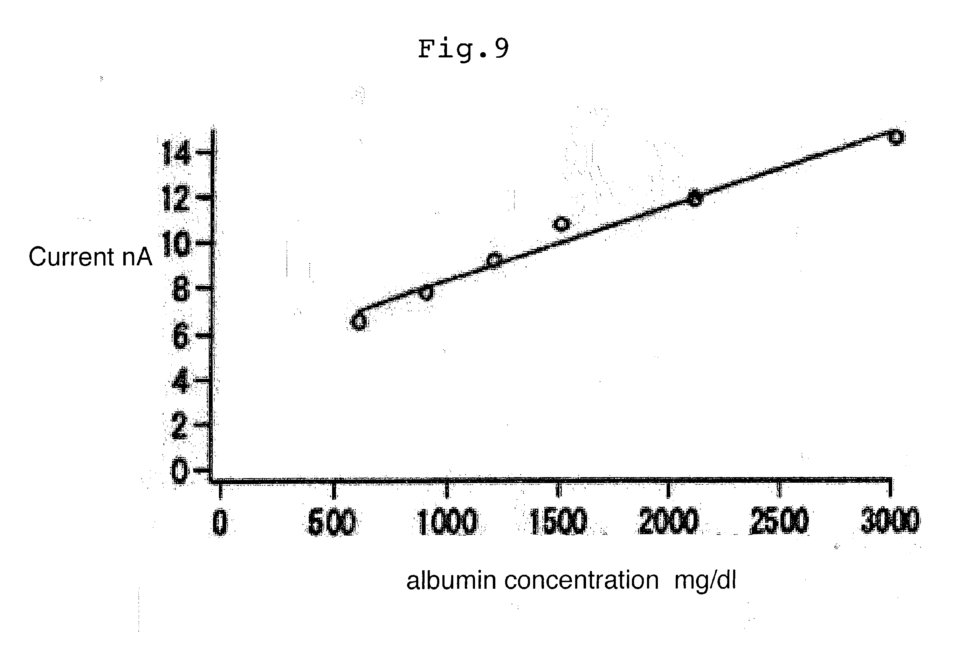

Fig. 9 shows a calibration curve showing a relationship between the concentration (horizontal axis) and the current value (vertical axis) of the BSA obtained by a flow injection analysis (hereinafter also called as FIA), wherein BSA is injected into 0.1 M phosphate buffer solution (pH7.0) at a constant rate that is made to contact with the electrode by the use of the boron dope diamond electrode as the working electrode 5, a platinum electrode as the counter electrode 4, and a silver/silver chloride electrode as the reference electrode 6. With the FIA, an S/B ratio is measured with changing a first impressed voltage from 0.4 - 1.0 V by 0.1 V, the S/B ratio becomes the largest at 0.8 V. Then, all of the measurements were conducted at 0.7 V. As a result, a linear calibration curve was obtained in a range wherein the concentration is from 5 mg/dl to 3000 mg/dl. With the FIA, since albumin is difficult to be absorbed into the working electrode, a good proportional relationship is obtained between the concentration of albumin and electric current as shown in the calibration curve. In addition, since the measurable range of the concentration is close to the range of the concentration of human blood, this method is one of the preferable measuring methods.

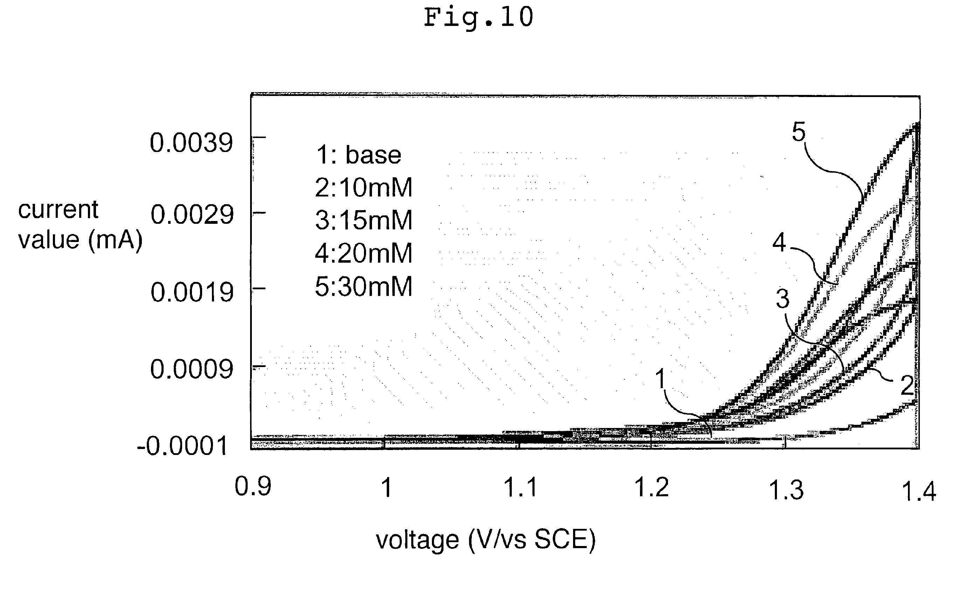

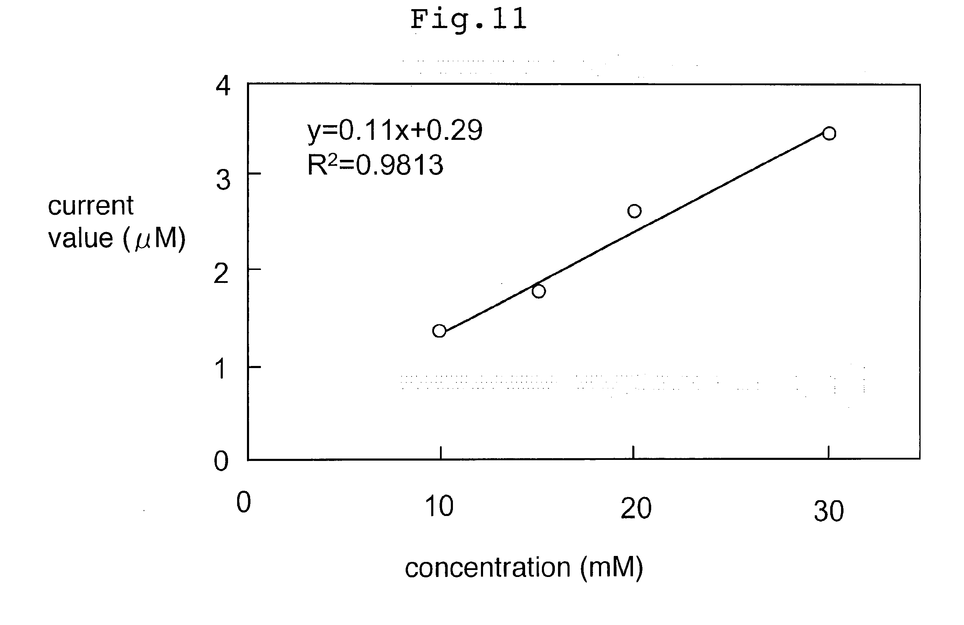

Fig. 10 shows a voltamogram with the cyclic voltammetry obtained by a linear curve sweeping (20 mV/s) with changing a concentration of tetrodotoxin in 0.1 M phosphate buffer solution (pH7.0) by the use of the boron dope diamond electrode as the working electrode 5, a platinum electrode as the counter electrode 4, and a SCE (saturated calomel electrode) as the reference electrode 6. In case that the object to be measured is tetrodotoxin, the maximum current value fell around 1.37 V. In addition, Fig. 11 shows a calibration curve showing a relationship between the concentration (horizontal axis) and the current value (vertical axis) obtained by measuring a tetrodotoxin solution having a known concentration by impressing a voltage of 1.37 V.

It has been already reported that a low-molecular substance can be detected by the use of the conductive diamond electrode. However, whether or not a high molecular weight substance such as a protein can be detected by the use of the conductive diamond electrode has never been known. As mentioned, it is the present claimed inventor who first found that the protein such as albumin, IAP, pepsin, hemoglobin and CRP or natural poison such as tetrodotoxin can be measured by the conductive diamond electrode.

In case of putting the measuring method of this invention into operation, a pretreatment operation for removing impurities contained in the sample solution 1 may be conducted. For example, a column process operation and a centrifugal separation operation may be represented as the pretreatment operation. If the pretreatment operation for removing the impurities is conducted, it is possible to further improve the measurement accuracy and the measurement sensitivity.

The present claimed invention is not limited to the above-mentioned embodiment and, for example, an electrochemical measuring device of a batch type may be used.

In addition, the electrochemical measuring device in accordance with this invention may comprise a separation device such as HPLC so that the object protein can be separated and refined and then the object protein fraction after being separated and purified can be analyzed.

### POSSIBLE APPLICATIONS IN INDUSTRY

As mentioned, in accordance with the present claimed invention, it is possible to detect various kinds of the protein and to measure its concentration at high speed with high accuracy and high sensitivity with a simple operation using a simple device.

## Claims

1. A method for measuring a protein that makes a working electrode and a counter electrode contact with a sample solution containing the protein as being an object to be measured, impresses a voltage between the working electrode and the counter electrode, measures an electric current value at this voltage and analyzes the protein electrochemically.

2. The method for measuring protein described in claim 1, wherein the protein is at least one protein selected from a group comprising albumin, immunosuppressive acidic protein, pepsin, hemoglobin and C-reactive protein.

3. The method for measuring a protein described in claim 1, wherein the working electrode is a conductive diamond electrode or a carbon electrode.

4. The method for measuring a protein described in claim 3, wherein
the conductive diamond electrode has a diamond thin film that is made to be conductive by doping group 13 elements or group 15 elements.

5. The method for measuring a protein described in claim 3, wherein
the conductive diamond electrode has a diamond thin film that is made to be conductive by doping at least one element selected from a group comprising,boron, nitrogen and phosphor.

6. The method for measuring a protein described in claim 3, wherein the conductive diamond electrode is a boron dope diamond electrode.

7. The method for measuring a protein described in claim 3, wherein
the conductive diamond electrode has a diamond thin film that is made to be conductive by doping one element that belongs to either one of group 8, group 9 and group 10.

8. The method for measuring a protein described in claim 3, wherein
the conductive diamond electrode has a diamond thin film that is made to be conductive by doping at least one element selected from a group comprising platinum, palladium, nickel, ruthenium and iridium.

9. The method for measuring a protein described in claim 1, wherein
a concentration of the protein as being the object to be measured in the sample solution is calculated from an electric current value by comparing a calibration curve prepared in advance between a concentration of the protein as being the object to be measured and the electric current value with the obtained electric current value.

10. The method for measuring a protein described in claim 1, wherein the voltage impressed between the working electrode and the counter electrode is a voltage that gives the maximum electric current value.

11. The method for measuring a protein described in claim 1, wherein an absolute value of the voltage impressed between the working electrode and the counter electrode is controlled by further making a reference electrode contact with the sample solution containing the protein as being the object to be measured.

12. The method for measuring a protein described in claim 3, and having a process to regenerate the conductive diamond electrode by means of cathodic reduction.

13. A device for measuring a protein that detects the protein as being an object to be measured in a sample solution, comprising
a cell that incorporates a working electrode and an counter electrode,
a device that impresses a voltage to the working electrode and the counter electrode,
a device that measures an electric current value at the impressed voltage, and
an information processing unit that detects the protein based on the obtained electric current value.

14. The device for measuring a protein described in claim 13, wherein
the working electrode is a conductive diamond electrode.

15. The device for measuring a protein described in claim 14, wherein
the conductive diamond electrode is a boron dope diamond electrode.

16. The device for measuring a protein described in claim 13, wherein
the cell further incorporates a reference electrode, and the device further comprises a device that controls an absolute value of the voltage impressed between the working electrode and the counter electrode.

17. The device for measuring a protein described in claim 14, comprising
a device that regenerates the conductive diamond electrode by means of cathodic reduction.

18. The device for measuring a protein described in claim 13, comprising
a separating device.

19. A method for measuring a protein,
comprising processes of making a carrier solution at a constant flow rate contact with a working electrode, injecting the protein as being an object to be measured into the carrier solution, making the carrier solution contact with the working electrode and a counter electrode, impressing a voltage between the working electrode and the counter electrode, measuring an electric current value at this voltage, and analyzing the protein electrochemically.
